# EUROPEAN PATENT APPLICATION

(11) **EP 4 715 041 A1**
(43) Date of publication of application: **25.03.2026**
(21) Application number: 24306550.5
(22) Date of filing: 20.09.2024
(51) Int. Cl.: C12N 5/071

(54) **HIGH-THROUGHPUT FABRICATION OF STRATIFIED CO-CULTURES**

(71) Applicant: L V M H RECHERCHE, 45800 St. Jean de Braye (FR); Ecole Polytechnique, 91120 Palaiseau (FR); Centre National de la Recherche Scientifique (CNRS), 75016 Paris (FR); Institut Pasteur, 75015 Paris (FR)
(72) Inventor: Vertti Quintero, Nadia, 45804 SAINT JEAN DE BRAYE CEDEX (FR); BULTEAU, Anne-Laure, 45800 SAINT JEAN DE BRAYE (FR); NIZARD, Carine, 45800 SAINT JEAN DE BRAYE (FR); LENZI, Elisa, 75015 Paris (FR); SART, Sébastien, 75015 Paris (FR); BAROUD, Charles, 91128 Palaiseau (FR)
(74) Representative: Regimbeau

(57) **Abstract**

The present invention concerns a method for preparing a spherical core-shell skin model, said method comprising the steps of:
a) forming microdroplets of an aqueous solution comprising fibroblasts and collagen, in an oil, wherein the formed collagen microdroplets comprise at least one fibroblast per microdroplet;
b) gelling at least a portion of the formed collagen microdroplets comprising said at least one fibroblast, thereby obtaining collagen beads comprising at least one fibroblast per bead;
c) replacing the oil surrounding the collagen beads comprising said at least one fibroblast with a culture medium,
d) contacting (i) the collagen beads comprising said at least one fibroblast with (ii) keratinocytes, and
e) incubating (i) said collagen beads with (ii) said keratinocytes for a period of time enabling the formation of an external layer by said keratinocytes around said collagen beads, thereby obtaining a spherical core-shell skin model, the core comprising at least one fibroblast and the shell comprising at least one keratinocyte.

## Description

### FIELD OF THE INVENTION

The present invention relates to three-dimensional skin models and their use in the screening of cosmetically, dermatologically or pharmaceutically active compounds.

### INTRODUCTION

Skin is composed of several cell types that interact with different extracellular matrix proteins (ECM) depending on their position within the tissue. Skin is usually described as being composed of three different stratified layers: epidermis, dermis and hypodermis. Epidermis, the outer layer of the skin is mainly composed of tightly interacting keratinocytes and limited ECM template. In contrast, the dermis positioned underneath the epidermis is composed of a dense ECM layer rich in collagens, glycosaminoglycans, hyaluronic acid, fibronectin, elastin (ELN), laminin, elastin fibers, and ground substances, which is mainly populated with fibroblasts playing the role of producers of this scaffolding material. Finally, the innermost layer is mainly composed of fat-storing adipocytes, and blood vessels with reduced ECM compounds.

To assess the effects of cosmetic ingredients on health, ageing processes or functional homeostasis of human skin, there is a need for *in vitro* models simulating the cellular and structural organization of the native tissue. This has led to the derivation of 3D skin models such as cultured tissue explants, reconstructed and bioprinted skin models. However, generating and maintaining these models is often demanding and resources consuming. Besides, the current 3D skin models often lack the possibility to parallelize their production to investigate several experimental conditions at the same time (*e.g*. for studying dose-response) or to get access to well-resolved cell population dynamics.

US application US2011/0306110 discloses the production of a 3D biomaterial comprising a gel layer which forms a core region where HepG2 cells (which are cells derived from liver cancer) are encapsulated and 3T3 fibroblast cells which cover around the gel layer. However, this US application does not suggest that such a 3D structure could be obtained from skin cells, nor that a 3D structure mimicking skin (with keratinocytes at the outside and fibroblasts in the inside) could be prepared.

There is thus a need for novel 3D skin models that would integrate the possibility of replicating the cellular complexity of the skin together with multiplexing experimental conditions.

### SUMMARY OF THE INVENTION

The present invention is based on the use of droplet microfluidics to fabricate spherical hydrogel microbeads that allow the co-culture of different cell types in a stratified manner at a very high frequency. The inventors indeed unexpectedly demonstrated that hydrogel microbeads comprising mesenchymal cells, namely fibroblasts, inside their inner part can be used as a template for the culture of epithelial cells, namely keratinocytes, on their surface, thereby allowing the co-culture of two different cell types, namely fibroblasts and keratinocytes, in a stratified manner, similar to skin organization.

More specifically, the present inventors showed that the method disclosed herein enables mimicking the structural organization of epidermis and dermis. Typically, and as detailed below, this method involves encapsulating a suspension of human fibroblasts (HFNs) into thousands of droplets of liquid collagen preferably using microfluidics devices. After gelation of the hydrogel droplets, the outer part of the collagen droplets containing HFNs is used as a support for culturing a keratinocyte cell line (HACATs). The inventors showed that the resulting 3D structures simulated the stratified organization of normal skin. Advantageously, their high-throughput production enabled multiplexing experimental conditions using them.

The present invention thus concerns a method for preparing a spherical core-shell skin model, said method comprising the steps of:
a) forming microdroplets of an aqueous solution comprising fibroblasts and collagen, in an oil, wherein the formed collagen microdroplets comprise at least one fibroblast per microdroplet;
b) gelling at least a portion of the formed collagen microdroplets comprising said at least one fibroblast, thereby obtaining collagen beads comprising at least one fibroblast per bead;
c) replacing the oil surrounding the collagen beads comprising said at least one fibroblast with a culture medium,
d) contacting (i) the collagen beads comprising said at least one fibroblast with (ii) keratinocytes, and
e) incubating (i) said collagen beads with (ii) said keratinocytes for a period of time enabling the formation of an external layer by said keratinocytes around said collagen beads, thereby obtaining a spherical core-shell skin model, the core comprising at least one fibroblast and the shell comprising at least one keratinocyte.

The present invention also concerns a spherical core-shell skin model obtainable by the preparation method of the invention.

Another object of the invention concerns a spherical core-shell skin model comprising:
- a core comprising fibroblasts in a collagen matrix, and
- a shell, totally or partially surrounding said core, the shell comprising keratinocytes forming an external layer on the outside of said core.

The present invention also concerns the use of the spherical core-shell skin model of the invention for screening cosmetically, dermatologically or pharmaceutically active candidate compounds.

Another object of the invention concerns a method for screening cosmetically, dermatologically or pharmaceutically active candidate compounds, said method comprising the steps of:
- providing at least one spherical core-shell skin model of the invention, and
- contacting said at least one spherical core-shell skin model with at least one cosmetically, dermatologically or pharmaceutically active candidate compound.

The present invention further concerns the use of the spherical core-shell skin model of the invention as a research tool for evaluating and/or monitoring the effect of external conditions on the expression level of skin cells proteins and/or on the mechanical properties of skin.

### DETAILED DESCRIPTION OF THE INVENTION

### Method of preparation of a spherical core-shell skin model

The present invention concerns a method for preparing a spherical core-shell skin model, said method comprising the steps of:
a) forming microdroplets of an aqueous solution comprising fibroblasts and collagen, in an oil, preferably at a temperature comprised between 2°C and 10°C, more preferably at a temperature of 4°C, wherein the formed collagen microdroplets comprise at least one fibroblast per microdroplet, preferably between 2 × 10⁶ and 3 × 10⁶ fibroblasts/mL of collagen microdroplet;
b) gelling at least a portion of the formed collagen microdroplets comprising said at least one fibroblast, thereby obtaining collagen beads comprising at least one fibroblast per bead, preferably between 2 × 10⁶ and 3 × 10⁶ fibroblasts/mL of collagen bead;
c) replacing the oil surrounding the collagen beads comprising said at least one fibroblast with a culture medium,
d) contacting (i) the collagen beads comprising said at least one fibroblast with (ii) keratinocytes, preferably at a ratio number of fibroblasts : number of keratinocytes comprised between 1:2 and 1:8, more preferably at a ratio number of fibroblasts : number of keratinocytes of 1:4, and
e) incubating (i) said collagen beads with (ii) said keratinocytes for a period of time enabling the formation of an external layer by said keratinocytes around said collagen beads, thereby obtaining a spherical core-shell skin model, the core comprising at least one fibroblast and the shell comprising at least one keratinocyte.

### Step a): formation of microdroplets

Step a) of the preparation method of invention comprises forming microdroplets of an aqueous solution comprising fibroblasts and collagen, in an oil, wherein the formed collagen microdroplets comprise at least one fibroblast per microdroplet.

As used herein, ***"microdroplets"*** refer to small, discrete volumes of fluids that are manipulated within immiscible phases typically using droplet-based microfluidics. More particularly, microdroplets are thus small, discrete volumes of liquid in a suspension, for example an emulsion, typically obtained using droplet-based microfluidics. Preferably, microdroplets refer to small, discrete volumes of aqueous liquids suspended in a non-aqueous liquid, such as an oil phase, typically obtained using droplet-based microfluidics.

In the context of the invention, microdroplets have a diameter of the order of a micrometer, preferably a diameter of between 10 and 1000 µm, typically a diameter comprised between 50 µm and 500 µm, more preferably a diameter comprised between 100 µm and 400 µm, most preferably a diameter comprised between 100 µm and 350 µm.

Preferably, each microdroplet has a volume comprised between 1 nL and 1 µL, more preferably a volume comprised between 1 nL and 100 nL, a volume comprised between 1 nL and 50 nL, most preferably a volume comprised between 10 nL and 20 nL. In a particularly preferred embodiment, each microdroplet has a volume of about 15 nL.

Microdroplets formed in step a) are microdroplets of an aqueous solution comprising fibroblasts and collagen.

By ***"aqueous solution"*** is meant herein a water solution. The aqueous solution may contain various solutes including but not limited to organic or inorganic salts, organic or inorganic acids, organic or inorganic bases, enzymes, proteins, nucleic acids, surfactants, and other organic or inorganic molecules.

In a particular embodiment, said aqueous solution is a culture medium.

In particularly preferred embodiment, said aqueous solution is a culture medium suitable for fibroblasts culture.

Culture media suitable for fibroblasts culture are well-known from the skilled person and include for example Dulbecco's Modified Eagle's Medium (DMEM), and MEM from Glasgow (GMEM)

In a particularly preferred embodiment, said aqueous solution is DMEM culture medium, typically supplemented with foetal bovine serum and/or antibiotics.

As used herein, a ***"fibroblast"*** refers to a cell of mesenchymal origin. Fibroblasts are found in connective tissue. They secrete the building blocks of the extracellular matrix, *i.e.* collagens, glycosaminoglycans, reticular and elastic fibers and glycoproteins.

In a particular embodiment, said fibroblast is a skin fibroblast. In a more particular embodiment, said fibroblast is a skin human fibroblast. In a particular embodiment, said fibroblast is a primary fibroblast, in particular a human primary fibroblast, more particularly a subject's derived human primary fibroblast.

In a particular embodiment, said fibroblasts are present in said aqueous solution at a concentration of 2 × 10⁶ cells/mL to 3 × 10⁶ cells/mL, more particularly at a concentration comprised between 2.1 × 10⁶ cells/mL and 2.9 × 10⁶ cells/mL, preferably between 2.2 × 10⁶ cells/mL and 2.8 × 10⁶ cells/mL, between 2.3 × 10⁶ cells/mL and 2.7 × 10⁶ cells/mL, between 2.3 × 10⁶ cells/mL and 2.6 × 10⁶ cells/mL, between 2.3 × 10⁶ cells/mL and 2.5 × 10⁶ cells/mL, or between 2.3 × 10⁶ cells/mL and 2.4 × 10⁶ cells/mL. In a particularly preferred embodiment, said fibroblasts are present in said aqueous solution at a concentration of 2.3 × 10⁶ cells/mL.

By ***"collagen"*** is meant herein the main structural protein in the extracellular matrix of connective tissues. Collagen includes type I collagen, type II collagen, type III collagen, type IV collagen and type V collagen.

In a preferred embodiment, said collagen is type I collagen.

By ***"type I collagen"*** is meant herein a fibrillar type collagen expressed in almost all connective tissues. Type I collagen molecules are typically heterotrimeric molecules with an α chain composition of two α1(I) chains and one α2(I) chain, typically encoded respectively by the *COL1A1* and *COL1A2* genes. This α chain composition is written as [α1(I)]₂α2(I).

In a particular embodiment, said collagen, in particular said type I collagen, is present in said aqueous solution at a concentration of 4 mg/mL to 10 mg/mL. In a particularly preferred embodiment, said collagen, in particular said type I collagen, is present in said aqueous solution at a concentration comprised between 4 mg/mL and 9 mg/mL, more particularly between 4 mg/mL and 8 mg/mL, more particularly between 4 mg/mL and 7 mg/mL, or between 5 mg/mL and 6 mg/mL. In a particularly preferred embodiment, said collagen, in particular said type I collagen, is present in said aqueous solution at a concentration of 5 mg/mL.

In a particular embodiment, said aqueous solution does not comprise any extracellular matrix component in addition to collagen.

In a particular embodiment, said aqueous solution does not comprise laminin and/or heparin sulphate proteoglycan.

By **"*oil*"** is meant herein a fat that is liquid at ambient temperature (25°C).

Examples of suitable oils include, but are not limited to, fluorinated oils, non-fluorinated oils, mineral oils, plant oils, vegetable oils, and comestible oils.

In some embodiments, the oil is an oil selected from the group consisting of a silicone oil, a fluorinated oil, a hydrocarbon, a paraffin oil, a mineral oil, a fatty acid oil, a plant oil and castor oil.

In a particular embodiment, the oil is a fluorinated oil.

By ***"fluorinated oil*"** is meant herein an oil comprising at least one added fluorine atom.

Examples of fluorinated oils include FC40 (typically commercialized by 3M^{™}), Pico-Wave^{™} 7500 (typically commercialized by Sphere Fluidics^{™}), Fluo-Oil 7500 or Fluo-Oil 40 (typically commercialized by Emulseo^{™}).

In particular embodiments, said fluorinated oil may be Novec^{™} 7500 Engineered Fluid (HFE) (typically commercialized by 3M^{™}).

Novec^{™} 7500 is hexane, 3-ethoxy-1,1,1,2,3,4,4,5,5,6,6,6-dodecafluoro-2-(trifluoromethyl) (also known as 2-(Trifluoromethyl)-3-ethoxydodecafluorohexane, CAS No. 297730-93-9):

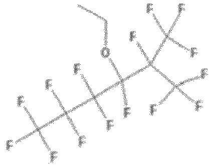

In a particular embodiment, said oil comprises a surfactant.

By ***"surfactant"*** is meant herein a surface-active compound that alters the surface tension that is exerted between each microdroplet and the oil phase, so as to stabilize the emulsion and avoid coalescence of the microdroplets.

As will be understood by the skilled person, the choice of surfactant will typically depend on the oil used.

Examples of surfactants include polyethylene glycol (PEG) tert-octylphenyl ether, sodium dodecylsulfate, cetyl PEG/polypropylene glycol (PPG) 10/1 dimethicone, fatty acid sorbitan esters, ethoxylated polysorbates, fatty acids, monolein, phospholipid, fatty alcohols, fluorinated surfactants, and combinations thereof.

In a particular embodiment, in particular when said oil is a fluorinated oil, said surfactant is a fluorinated surfactant.

By ***"fluorinated surfactant"*** is meant herein an amphiphilic compound suitable to stabilize the microdroplets in the oil phase, comprising a hydrophilic moiety and a hydrophobic moiety, said hydrophobic moiety comprising fluorine atoms (i.e. a fluorocarbon part).

Examples of fluorinated surfactant include Picosurf^{®} (commercialized by Sphere Fluidics^{™}), such as Picosurf^{™} 1 and Picosurf^{™} 2, 008-FluoroSurfactant (commercialized by RAN Biotechnologies, Krytox^{™} 157 (commercialized by Chemours), and FluoSurf (from Emulseo).

In a particular embodiment, said fluorinated surfactant is 008-FLuoroSurfactant (commercialized by RAN Biotechnologies).

In a particular embodiment, said fluorinated surfactant is present in said oil, in particular in said fluorinated oil, at a concentration of 2% w/v to about 5% w/v, more particular of 3% w/v to 4% w/v.

In a preferred embodiment, step a) of forming microdroplets is carried out at a temperature comprised between 2°C and 10°C, more particularly between 2°C and 9°C, between 2°C and 8°C, between 2°C and 7°C, between 3°C and 6°C, or between 4°C and 5°C.

In a particularly preferred embodiment, step a) of forming microdroplets is carried out at a temperature of 4°C.

The collagen microdroplets formed in step a) comprise at least one fibroblast per microdroplet. Preferably, the collagen microdroplets formed in step a) comprise between 2 × 10⁶ and 3 × 10⁶ fibroblasts/mL of collagen microdroplet, more preferably between 2.1 × 10⁶ and 2.9 × 10⁶ fibroblasts/mL of collagen microdroplet, between 2.2 × 10⁶ and 2.8 × 10⁶ fibroblasts/mL of collagen microdroplet, between 2.3 × 10⁶ and 2.7 × 10⁶ fibroblasts/mL of collagen microdroplet, between 2.3 × 10⁶ and 2.6 × 10⁶ fibroblasts/mL of collagen microdroplet, between 2.3 × 10⁶ and 2.5 × 10⁶ fibroblasts/mL of collagen microdroplet, or between 2.3 × 10⁶ and 2.4 × 10⁶ fibroblasts/mL of collagen microdroplet. In a particularly preferred embodiment, the collagen microdroplets formed in step a) comprise 2.3 × 10⁶ fibroblasts/mL of collagen microdroplet.

Therefore, when the collagen microdroplets have a diameter of 100 µm to 500 µm, in particular a diameter of 100 µm to 350 µm, the collagen microdroplets formed in step a) preferably comprise between 10 and 100 fibroblasts per collagen microdroplet, more preferably between 25 and 50 fibroblasts per collagen microdroplet. In a particularly preferred embodiment, when the collagen microdroplets have a diameter of 100 µm to 350 µm, the collagen microdroplets formed in step a) comprise about 40 fibroblasts per collagen microdroplet.

As used herein, the term ***"about"*** or ***"approximately"*** refers to the normal range of error for a given value or range known to the person of skills in the art. It usually means within 20%, such as within 10%, or within 5% (or 1% or less) of a given value or range.

In a particular embodiment, step a) of forming microdroplets is implemented using a microfluidic device.

By ***"microfluidic device"*** is meant herein a device, the parts of which are manufactured according to microfabrication processes. Such a device comprises channels, at least one dimension of which is typically less than a millimeter.

Numerous methods have already been proposed to form microdroplets in a continuous phase such as oil. For example, mention may be made of the following examples of methods:
- the method referred to as "flow-focusing", described for example in Anna et al. (2003) Appl. Phys. Lett. 82:364, the content of which is incorporated herein by reference,
- the "T-junction" method, described for example in Thorsen et al. (2001) Phys. Rev. Lett. 86:4163-4166, the content of which is incorporated herein by reference, or else
- the "confinement gradient" method described for example in the application FR2958186, the content of which is incorporated herein by reference.

These methods make it possible to form microdroplets with substantially equal dimensions.

In a preferred embodiment, said microdroplets are formed using the "flow-focusing" method.

In a particular embodiment, said microfluidic device is a microfluidic chip.

By ***"microfluidic chip"*** is meant herein an independent integrated unit comprising a microfluidic chamber and one or more microfluidic channels. The microfluidic chip can also be connected to pumps or pressure-based flow controllers.

Said microfluidic device or chip can be microfabricated by any technique well-known in the art. For example, said microfluidic device or chip can be prepared using molds that have been designed for the particular use of said microfluidic device or chip. Said molds may be made in any suitable material, such as dry resin, for example dry resin Eternal Laminar E8013, commercialized by Eternal materials.

The different parts of said microfluidic device or chip, in particular the parts prepared from molds as disclosed above, can be in any suitable material, such as poly(dimethylsiloxane) (PDMS). The different parts of said microfluidic device or chip can then be bonded on an appropriate surface, such as a glass slide, using any suitable technique, such as oxygen plasma bonding technique.

Before being used to form the microdroplets of the invention, the microfluidic device or chip can be surface-treated, typically with a coating agent, suitable to be used with the oil used to form said microdroplets. In a particular embodiment, said microfluidic device or chip is surface-treated, before use, with a fluoropolymer surface agent, such as Novec^{™}-1720, commercialized by 3M^{™}.

In a particular embodiment, step a) of the preparation method of the invention is implemented using a microfluidic device or chip as defined above and comprises:
a1) injecting, into a zone of said microfluidic device or chip, the aqueous solution, as defined above, comprising fibroblasts, as defined above, and collagen, as defined above, in particular type I collagen, as defined above
a2) injecting the oil, as defined above, into said zone of said microfluidic device or chip, in order to drive the aqueous solution comprising fibroblasts and collagen, in particular type I collagen, towards an outlet of said microfluidic device or chip, the oil being injected so as to form collagen microdroplets comprising at least one fibroblast per microdroplet, preferably between 2 × 10⁶ and 3 × 10⁶ fibroblasts/ml of collagen microdroplet, and
a3) collecting, from said microfluidic device or chip, said formed collagen microdroplets comprising at least one fibroblast per microdroplet.

By ***"zone of the microfluidic device (or chip)"*** is meant herein a part of said microfluidic device (or chip), typically prepared from a mold, designed to obtain microdroplets of a given size.

By ***"outlet of the microfluidic device (or chip)"*** is meant herein the openings where fluid exits the microfluidic device (or chip).

By ***"injection"*** is meant herein the introduction, under pressure, of a liquid into a zone.

In a particular embodiment, said aqueous solution and/or said oil is(are) injected using a syringe, typically connected to inlets of the microfluidic device or chip, preferably through tubing, in particular through polytetrafluoroethylene (PTFE) tubing.

By ***"inlet of the microfluidic device (or chip)"*** is meant herein the openings where fluid enters the microfluidic device (or chip).

In a particular embodiment, said microfluidic device or chip is filled with oil, as defined above, before use, in particular before injection of the aqueous solution.

In a particular embodiment, said syringes are filled with oil, as defined above, before use, in particular before being loaded with the aqueous solution.

Said aqueous solution and said oil can be injected into said zone of said microfluidic device or chip at any suitable flow rate. As will be understood by the skilled person, the choice of the flow rate of injection of the aqueous solution and of the oil will depend on the size of the microdroplets to be formed.

In a particular embodiment, said aqueous solution is injected into said zone of said microfluidic device or chip at a flow rate comprised between 1 µL/min and 10 µL/min, more particularly between 2 µL/min and 9 µL/min, between 3 µL/min and 8 µL/min, between 4 µL/min and 7 µL/min or between 5 µL/min and 6 µL/min. In a particularly preferred embodiment, said aqueous solution is injected into said zone of said microfluidic device or chip at a flow rate of 5 µL/min.

In a particular embodiment, said oil is injected into said zone of said microfluidic device or chip at a flow rate comprised between 10 µL/min and 100 µL/min, more particularly between 20 µL/min and 90 µL/min, between 30 µL/min and 80 µL/min, between 40 µL/min and 70 µL/min or between 50 µL/min and 60 µL/min. In a particularly preferred embodiment, said oil is injected into said zone of said microfluidic device or chip at a flow rate of 50 µL/min.

The collecting step of the formed collagen microdroplets can be carried out in any collecting device such as an Eppendorf tube.

As will be understood by the skilled person, the formed collagen microdroplets will be collected within oil used for their formation.

### Step b): gelling of microdroplets

Step b) of the preparation method of the invention comprises gelling at least a portion of the formed collagen microdroplets comprising said at least one fibroblast as defined above in the section "Step a): formation of microdroplets", thereby obtaining collagen beads comprising at least one fibroblast per bead.

By ***"gelling"*** is meant herein turning into gel under specific conditions such as the internal composition or/and the action of external stimuli.

As used herein, the term ***"gel"*** or ***"gelled"*** refers to the state of matter between liquid and solid. As such, a "gel" has some of the properties of a liquid (i.e., the shape is resilient and deformable) and some of the properties of a solid (i.e., the shape is discrete enough to maintain three dimensions on a two-dimensional surface).

In a particular embodiment, said gelling step b) comprises heating said formed collagen microdroplets.

Preferably, the formed collagen microdroplets are heated at a temperature higher than 10°C, more preferably higher than 20°C, higher than 25°C, higher than 30°C or higher than 35°C, most preferably at a temperature of 37°C.

Said heating can be performed by any technique well-known from the skilled person.

Preferably, said heating is performed during at least 1 min, preferably during at least 5 min, more preferably during at least 10 min.

By ***"at least a portion"*** is meant herein that at least 10%, more preferably at least 20%, at least 30%, at least 40%, at least 50%, at least 60%, at least 70%, at least 80%, at least 90%, at least 95%, at least 96%, at least 97%, at least 98%, at least 99% or 100% of the formed collagen microdroplets are gelled.

The thereby obtained collagen beads typically have a diameter of 50 µm to 500 µm, more preferably a diameter comprised between 100 µm and 400 µm, most preferably a diameter comprised between 100 µm and 350 µm.

The collagen beads obtained at the end of step b) display a collagen fiber mesh within which fibroblasts are present and remodel collagen organization overtime.

The thereby obtained collagen beads comprise at least one fibroblast per bead, more preferably between 2 × 10⁶ and 3 × 10⁶ fibroblasts/mL of collagen bead, more preferably between 2.1 × 10⁶ and 2.9 × 10⁶ fibroblasts/mL of collagen bead, between 2.2 × 10⁶ and 2.8 × 10⁶ fibroblasts/mL of collagen bead, between 2.3 × 10⁶ and 2.7 × 10⁶ fibroblasts/mL of collagen bead, between 2.3 × 10⁶ and 2.6 × 10⁶ fibroblasts/mL of collagen bead, between 2.3 × 10⁶ and 2.5 × 10⁶ fibroblasts/mL of collagen bead, or between 2.3 × 10⁶ and 2.4 × 10⁶ fibroblasts/mL of collagen bead. In a particularly preferred embodiment, the thereby obtained collagen beads comprise 2.3 × 10⁶ fibroblasts/mL of collagen bead.

Therefore, when the collagen beads have a diameter of 100 µm to 500 µm, in particular a diameter of 100 µm to 350 µm, the collagen beads obtained in step b) preferably comprise between 10 to 100 fibroblasts per collagen bead, more preferably between 25 and 50 fibroblasts per collagen bead. In a particularly preferred embodiment, when the collagen beads have a diameter of 100 µm to 350 µm, the collagen beads obtained in step b) comprise about 40 fibroblasts per collagen bead.

### Step c): replacement of oil

Step c) of the preparation method of the invention comprises replacing the oil surrounding the collagen beads comprising said at least one fibroblast with a culture medium.

Said culture medium can be the same as the one defined above in the section "Step a): formation of microdroplets".

In a particular embodiment, said culture medium is a culture medium suitable for keratinocytes culture.

Culture media suitable for keratinocytes culture are well-known from the skilled person and include for example Dulbecco's Modified Eagle's Medium (DMEM), or Epi-life.

In a particular embodiment, said culture medium is DMEM culture medium, typically supplemented with foetal bovine serum and/or antibiotics.

In a particular embodiment, the method of invention comprises before the replacing step c), a step of cleaning the gelled collagen beads from oil.

By **"*cleaning the beads from oil*"** is meant herein removing at least part, preferably the totality, of the oil present at the interface with the collagen beads.

Said cleaning step can be performed by any technique well-known from the skilled person.

In particular, when the oil used to form collagen microdroplets comprises a surfactant, said cleaning step can first comprise incubating the collagen beads in said oil without surfactant.

In a particular embodiment, said cleaning step further comprises contacting the collagen beads with an emulsion destabilization agent.

By ***"emulsion destabilization agent"*** is meant herein an agent able to compete with 008-FluoroSurfactant (commercialized by RAN Biotechnologies).

Examples of emulsion destabilization agents are well-known from the skilled person and include: 1H,1H-perfluoro-1-pentanol; 1H,1H-perfluoro-1-octanol; 1H,1H,8H-perfluoro-1-octanol; and 1H,1H,2H,2H-perfluoro-1-octanol.

In a particular embodiment, said emulsion destabilization agent is 1H,1H,2H,2H-perfluoro-1-octanol (PFO), preferably PFO dissolved in oil, as defined in the section "Step a): formation of microdroplets".

At the end of the replacement step, the collagen beads comprising said at least one fibroblast are suspended in said culture medium.

### Step d): contacting of the collagen beads with keratinocytes

Step d) of the preparation method of the invention comprises contacting (i) the collagen beads comprising said at least one fibroblast with (ii) keratinocytes.

By *"keratinocyte"* is meant herein a cell which is found in the epidermis and produces keratin.

In a particular embodiment, said keratinocyte is a human keratinocyte, preferably an immortalized human keratinocyte such as a keratinocyte from the HACAT cell line.

Preferably, said contacting of (i) the collagen beads comprising said at least one fibroblast with (ii) keratinocytes is performed at a ratio number of fibroblasts : number of keratinocytes comprised between 1:2 and 1:8, more preferably between 1:2 and 1:7, between 1:3 and 1:6 or between 1:4 and 1:5. Most preferably, said contacting of (i) the collagen beads comprising said at least one fibroblast with (ii) keratinocytes is performed at a ratio number of fibroblasts : number of keratinocytes of 1:4.

Preferably said keratinocytes are in the form of a suspension in a culture medium, in particular in a culture medium suitable for keratinocytes culture as defined in the section "Step c): replacement of oil" above.

In the context of the invention, the keratinocytes suspension does not comprise any gelling agent.

In a preferred embodiment, in step d), the collagen beads (i) are contacted with a keratinocytes suspension (ii) at a concentration of 4 × 10¹ keratinocytes/mL to 5 × 10¹ keratinocytes/mL, preferably at a concentration of 4.1 × 10¹ keratinocytes/mL to 4.9 × 10¹ keratinocytes/mL, at a concentration of 4.2 × 10¹ keratinocytes/mL to 4.8 × 10¹ keratinocytes/mL, at a concentration of 4.3 × 10¹ keratinocytes/mL to 4.7 × 10¹ keratinocytes/mL, at a concentration of 4.4 × 10¹ keratinocytes/mL to 4.6 × 10¹ keratinocytes/mL, most preferably at a concentration of 4.5 × 10¹ keratinocytes/mL.

In a particular embodiment, said contacting step d) is performed under low agitation to allow homogenous distribution of keratinocytes on the collagen beads.

By ***"low agitation"*** is meant herein an agitation lower than 50 rpm (rotation per minute), more preferably an agitation lower than 40 rpm, lower than 30 rpm, lower than 20 rpm, more preferably lower 10 rpm.

In a particular embodiment, said contacting step d) is performed under agitation at 8 rpm.

By ***"homogenous distribution"*** is meant herein the uniform distribution of the keratinocytes on the surface of the collagen beads. ***"Homogenous distribution"*** also refers to a similar amount of keratinocytes on the surface of each collagen bead.

Preferably, said contacting step is performed at a temperature suitable for fibroblasts and/or keratinocytes culture, in particular at 37°C.

Preferably, said contacting step is performed for at least 30 min, more preferably for at least 1 h.

### Step e): incubation

Step e) of the preparation method of the invention comprises incubating (i) said collagen beads with (ii) said keratinocytes for a period of time enabling the formation of an external layer by said keratinocytes around said collagen beads, thereby obtaining a spherical core-shell skin model, the core comprising at least one fibroblast and the shell comprising at least one keratinocyte.

By ***"incubating"*** or ***"incubation"*** is meant herein maintaining under conditions favorable for cell culture and/or cell survival.

In particular, said incubation step enables keratinocytes multiplication, so as to form a cell layer.

By ***"layer"*** is meant herein a layer of any thickness comprising one or more keratinocyte cells. In the context of the invention, said layer may be a monolayer of keratinocytes or a multilayer of keratinocytes.

By ***"external layer"*** is meant herein that said keratinocytes form a monolayer or a multilayer of cells at the external side of the collagen beads.

As will be understood by the skilled person, since the keratinocyte suspension used to form the external layer of keratinocytes does not comprise any gelling agent, said external layer is not in the form of a gel.

As used herein, the period of time enabling the formation of an external layer of said keratinocytes around the collagen beads is the period of time needed by the keratinocytes to multiply to form such a layer. As will be understood by the skilled person, this period of time will depend, among others, on the initial number of keratinocytes, the culture medium used and the culture temperature.

In a particular embodiment, said incubation step e) is performed for at least 12h, preferably of at least 1 day, most preferably of 1 day to 3 days.

The inventors indeed demonstrated that by incubating the collagen beads with the keratinocytes for at least 12h, preferably for at least 1 day, the fibroblasts comprised in the collagen beads were enabled to reorganize fibers in the collagen matrix and/or the keratinocytes were enabled to form an external layer as defined above on the outside of said collagen beads.

Therefore, in a particular embodiment, said incubation step e) is performed for at least one day, thereby enabling fibroblasts to reorganize fibers in the collagen matrix and keratinocytes to form an external layer on the outside of said collagen beads.

By ***"reorganize fibers in the collagen matrix"*** is meant herein that said fibroblasts induce the spatial modification of the collagen matrix.

As shown in the examples below, this reorganization of the fibers in the collagen matrix leads to a decrease in the diameter of the beads. Therefore, in a preferred embodiment the spherical core-shell skin model obtained at the end of step e) has a diameter comprised between 100 µm and 200 µm.

This incubation step thus enables the formation of a spherical core-shell skin model, the core comprising at least one fibroblast as defined above and the shell comprising at least one keratinocyte as defined above.

By ***"spherical skin model"*** is meant herein that said skin model is in 3 dimensions (3D) and displays an essentially spherical form.

By ***"core-shell skin model"*** is meant herein that said skin model is composed of one compartment, the core, surrounded by a shell.

As will be understood by the skilled person, the core compartment is mainly composed of the collagen matrix, in particular the reorganized collagen matrix, and the fibroblasts, while the shell surrounding the core, is mainly composed of a monolayer or multilayer of keratinocytes. In the context of the invention, said shell is not in the form of a gel.

### Spherical core-shell skin model

The present invention also concerns a spherical core-shell skin model obtainable by the method of preparation as defined in the section *"Method of preparation of a spherical core-shell skin model"* above.

The present invention also concerns a spherical core-shell skin model comprising:
- a core comprising fibroblasts, as defined in the section *"Method of preparation of a spherical core-shell skin model"* above, in a collagen, in particular a type I collagen, matrix, as defined in the section *"Method of preparation of a spherical core-shell skin model"* above, and
- a shell, totally or partially surrounding said core, the shell comprising keratinocytes, as defined in the section *"Method of preparation of a spherical core-shell skin model"* above, forming an external layer, as defined in the section *"Method of preparation of a spherical core-shell skin model"* above, on the outside of said core.

As used herein, a spherical core-shell skin model has the same definition as the one provided in the section *"Method of preparation of a spherical core-shell skin model"* above.

The spherical core-shell skin model of the invention is particularly advantageous because of its specific stratified 3D structure. Indeed, the keratinocytes being at the outside of the model, they are located at the interface with the external environment and can be directly contacted with an external compound or stimulus, similarly to what happens on skin. On the contrary, the fibroblasts are at the inside of the model within a collagen matrix, separated from the external environment by the keratinocyte layer. Therefore, an effect of an external compound or stimulus on the fibroblasts will necessarily be mediated by the keratinocyte layer, similarly to what happens on skin. The spherical core-shell skin model of the invention is therefore a very interesting mimic of the skin interface.

As used herein, the term *"shell totally surrounding the core"* means that the shell formed by the layer(s) of keratinocytes surrounds essentially the totality of the core surface formed by the fibroblasts/collagen matrix assembly.

As used herein, the term ***"essentially"*** means "apart from parts that are insignificant in the context of the invention". Therefore, a shell surrounding essentially the totality of the core surface means that the shell surrounds the whole core surface except possibly for small non-significant zone(s) that typically represent(s) less than 5% of the total core surface. In other words, a shell surrounding essentially the totality of the core surface surrounds more than 95% of the total core surface, more preferably more than 99% of the total core surface.

As used herein, the term ***"shell partially surrounding the core"*** means that the shell formed by the layer(s) of keratinocytes only surrounds a portion of the core surface formed by the fibroblasts/collagen matrix assembly. Preferably, said portion of the core surface represents between 50% and 95% of the total core surface, more preferably between 60% and 95%, between 70% and 95%, between 80% and 95%, or between 90% and 95% of the total core surface.

In the context of the invention, said shell is not in the form of a gel.

Preferably, said external layer of keratinocytes is a double layer, or more, of keratinocytes.

In a preferred embodiment, the spherical core-shell skin model preferably has a diameter of between 10 and 1000 µm, typically a diameter comprised between 50 µm and 500 µm, more preferably a diameter comprised between 100 µm and 400 µm, more preferably a diameter comprised between 100 µm and 350 µm, most preferably a diameter comprised between 100 µm and 200 µm.

In a particular embodiment, the spherical core-shell skin model has a stiffness of more than 2 kPa.

As used herein, the term ***"stiffness"*** refers to the elastic modulus (also known as Young's modulus) of a material, i.e., a measure of a material's resistance to being deformed elastically when a force is applied to the material.

Young's modulus value of the spherical core-shell skin model can be determined by any technique well-known from the skilled person, such as by microindentation. Typically, said Young's modulus value is determined using micropipettes or atomic force microscopy (AFM).

The inventors demonstrated that the stiffness value of the spherical core-shell skin model, which is within the same range as the skin stiffness value, is due to the combined presence, in the core of the model, of the collagen matrix and the fibroblasts. Indeed, without willing to be bound by theory, the remodeling of the collagen fibers induced by the fibroblasts increases the rigidity of the model compared to a model without fibroblasts.

The inventors further demonstrated that the spherical core-shell skin model of the invention displays a structural organization similar to the skin's organization, which is maintained overtime, in particular up to 7 days after the preparation of the model.

The inventors also demonstrated that the keratinocytes of the spherical core-shell skin model of the invention express key skin phenotypic markers, which is not the case for example of some non-three-dimensional models.

Therefore, in a particular embodiment, in the spherical core-shell skin model of the invention, said keratinocytes display a higher expression level of collagen VII, involucrin, loricrin, keratin 1 and/or keratin 10 compared to the same cells cultured, alone or in co-culture with fibroblasts, in two-dimensions.

As used herein, the term ***"collagen VII"*** or ***"type VII collagen"*** refers to a non-fibrillar collagen which is the main constituent of anchoring fibrils, which in the skin are located below the basal lamina at the dermal-epidermal basement membrane zone. Collagen VII is typically encoded by the *COL7A1* gene. Human collagen VII typically comprises the amino acid sequence referenced under Uniprot database number Q02388.

As used herein, the term ***"involucrin"*** refers to a protein component of human skin which is typically encoded by the *IVL* gene. In binding the protein loricrin, involucrin contributes to the formation of a cell envelope that protects corneocytes in the skin. Human involucrin typically comprises the amino acid sequence referenced under Uniprot database number P07476.

As used herein, the term ***"loricrin"*** refers to a glycine-serine-cysteine-rich insoluble protein, synthesized in stratum granulosum, which is typically encoded by the *LOR* gene. Human loricrin typically comprises the amino acid sequence referenced under Uniprot database number P23490.

As used herein, the term ***"keratin*** 1" refers to a Type II intermediate filament of the intracytoplasmatic cytoskeleton, which is co-expressed with and binds to Keratin 10, a Type I keratin, to form a coiled coil heterotypic keratin chain. Keratin 1 is typically encoded by the *KRT1* gene. Human keratin 1 typically comprises the amino acid sequence referenced under Uniprot database number P04264.

As used herein, the term ***"keratin 10"*** refers to a member of the type I (acidic) cytokeratin family, which composes the cytoskeleton of epithelial cells. Keratin 10 is typically encoded by the *KRT10* gene. Human keratin 10 typically comprises the amino acid sequence referenced under Uniprot database number P13645.

By ***"expression level"*** is meant herein a level of expression of a gene, which can be determined by usual method known from the skilled person. In particular, each expression level may be measured at the genomic and/or nucleic and/or protein level. In a preferred embodiment, the expression level is determined by measuring the amount of nucleic acid transcripts of the gene, such as PCR, quantitative PCR (qPCR), NGS (Next-Generation Sequencing (NGS)) and RNA sequencing. In another embodiment, the expression level is determined by measuring the amount of protein produced by the gene.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-known in the art. From the mRNA or cDNA sample, the amount of nucleic acid transcripts may be measured using any technology known by the skilled person, including nucleic microarrays, quantitative PCR, next generation sequencing and hybridization with a labelled probe.

In particular, the level of mRNA expression for the gene may be performed by the well-known techniques of the skilled in the art such as hybridization technique and/or amplification technique (PCR), using suitable primers or probes that are specific for the gene mRNA.

Illustratively, mRNA may be extracted, for example using lytic enzymes or chemical solutions or extracted by commercially available nucleic-acid-binding resins following the manufacturer's instructions. Extracted mRNA may be subsequently detected by hybridization, such as Northern blot, and/or amplification, such as quantitative or semiquantitative RT-PCR.

The amount of mRNA can be measured by any technology known by a person skilled in the art, including mRNA microarrays, quantitative PCR, next generation sequencing and hybridization with a labelled probe. In particular, real time quantitative RT-PCR (qRT-PCR) may be useful. In some embodiments, qRT-PCR can be used for both the detection and quantification of RNA targets. Commercially available qRT-PCR based methods (*e.g*.,Taqman^{®} Array) may for instance be employed, the design of primers and/or probe being easily made based on the sequence of the genes.

mRNA assays or arrays can also be used to assess the levels of the mRNAs in a sample.

Alternatively, the level of protein expression for the gene may be measured by well-known techniques including detection and quantification of the protein of interest by the means of any type of ligand molecule that specifically binds thereto, including nucleic acids (for example nucleic acids selected for binding through the well-known SELEX method), antibodies and antibody fragments. The antibodies to said given protein of interest may be easily obtained with conventional techniques, including generation of antibody-producing hybridomas.

*In vitro* techniques for detection of a protein of interest include enzyme linked immunosorbent assays (ELISAs), Western blots, immunoprecipitations and immunofluorescence.

By ***"higher expression level"*** is meant herein a a statistically significant increase in the expression level of the gene of interest with respect to the control level, *i.e.* the same cells cultured, alone or in co-culture with fibroblasts, in two-dimensions.

By ***"culture in 2-dimensions"*** is meant herein that said cells are cultured in a 2-dimensions environment.

A culture in 2-dimensions is typically a culture in Petri dishes, flasks, or microwell plates. In a particular embodiment, the culture in 2-dimensions is a culture in flasks.

By ***"culture alone"*** is meant herein that the keratinocytes are the only cell type cultured in the culture container, the cells having no direct or indirect contact with another cell type.

By ***"co-culture with fibroblasts"*** is meant herein the keratinocytes are cultured in the presence of fibroblasts, preferably from the same type as the fibroblasts comprised in the spherical core-shell skin model.

### Screening of cosmetically, dermatologically or pharmaceutically active compounds - Research tool

The present invention also concerns the use of the spherical core-shell skin model as defined in the section *"Spherical core-shell skin model"* above for screening cosmetically, dermatologically or pharmaceutically active candidate compounds.

Another object of the invention concerns a method for screening cosmetically, dermatologically or pharmaceutically active candidate compounds, said method comprising the steps of:
- providing at least one spherical core-shell skin model as defined in the section *"Spherical core-shell skin model"* above, and
- contacting said at least one spherical core-shell skin model with at least one cosmetically, dermatologically or pharmaceutically active candidate compound.

As explained above, the spherical core-shell skin model of the invention displays a structural organization similar to the skin's organization, and express key skin phenotypic markers. It can thus be advantageously used to screen compounds that may be cosmetically, dermatologically or pharmaceutically active on skin.

By ***"cosmetically active compound"*** is meant herein a compound, substance or molecule exerting a cosmetic action on the surface of the animal or human body. Said cosmetic action can be any action relevant for the care and make-up of the skin, such as dyeing or coloring, cosmetic care-imparting, conditioning, protective, hardening, softening or moisturizing action.

By ***"dermatologically active compound"*** is meant herein a compound, substance or molecule exerting a therapeutic or prophylactic action on animal or human skin affected by a skin disease. Said action can be any action relevant for the health of the skin, in particular for treating a skin disease.

Examples of skin diseases include psoriasis, acne, alopecia areata, basal cell carcinoma, melanoma, Bowen's disease, contact dermatitis, Darier's disease, atopic eczema, hailey-hailey disease, herpes simplex, hidradenitis suppurativa, hirsutism, hyperhidrosis, ichthyosis, impetigo, keloids, keratosis pilaris, lichen planus, lichen sclerosus, melasma, verrucas, pityriasis lichenoides, polymorphic light eruption, pyoderma gangrenosum, rosacea, scabies, shingles, squamous cell carcinoma, vitiligo, premature skin aging, and photodamage.

By ***"pharmaceutically active compound"*** is meant herein a compound, substance or molecule exerting a therapeutic or prophylactic action on animal or human skin.

The spherical core-shell skin model of the invention is particularly advantageous for the screening of cosmetically, dermatologically or pharmaceutically active candidate compounds because, since it can be produced quickly and in high quantity, it can be used in high-throughput methods to screen multiple candidate compounds at the same time and/or to screen multiple doses of candidate compound at the same time and/or to screen a candidate compound in multiple replicates at the same time.

In a particular embodiment, said at least one spherical core-shell skin model is contacted with said at least one cosmetically, dermatologically or pharmaceutically active candidate compound by any method well-known from the skilled person, such as by incubating said spherical core-shell skin model in a solution or culture medium comprising said cosmetically, dermatologically or pharmaceutically active candidate compound(s).

In a particular embodiment, said screening method further comprises, after said contacting step, a step of measuring parameter(s) and/or biomarker(s) of interest in the cells or collagen matrix of the spherical core-shell skin model(s), and optionally comparing the measured parameter(s) and/or biomarker(s) with those measured in a spherical core-shell skin model which has not been contacted with said cosmetically, dermatologically or pharmaceutically active candidate compound(s).

In a particular embodiment, said screening method is implemented using a microfluidic device or chip, as defined in the section *""Method of preparation of a spherical core-shell skin model"* above.

In a particular embodiment, said spherical core-shell skin model can be used to screen cosmetically, dermatologically or pharmaceutically active compounds in the context of personalized medicine or care.

Therefore, in a particular embodiment, said keratinocytes comprised in the shell of said spherical core-shell skin model, and optionally said fibroblasts comprised in the core of said spherical core-shell skin model, are obtained from a subject or patient to whom said cosmetically, dermatologically or pharmaceutically active compounds is planned to be administered.

The present invention also concerns the use of the spherical core-shell skin model as defined in the section *"Spherical core-shell skin model"* above, as a research tool for evaluating and/or monitoring the effect of external conditions on the expression level of skin cells proteins, in particular the expression level of proteins from skin fibroblasts and/or keratinocytes, and/or on the physiological and/or mechanical properties of skin.

By ***"external condition"*** is meant herein external factors likely to be applied to skin, such as environmental stress, temperature, ultraviolet radiation, environmental pollution, accelerated ageing conditions, microbiota and the like.

By ***"physiological properties of skin"*** is meant herein the characteristics of skin that are related to its functioning and performance, which typically include physical barrier property, immune barrier property, and the like.

By ***"mechanical properties of skin"*** is meant herein the mechanical characteristics of skin in various conditions and under diverse external loads, and typically include tensile strength, stiffness and extensibility.

The present invention will be further illustrated by the figures and examples below.

### BRIEF DESCRIPTION OF THE FIGURES

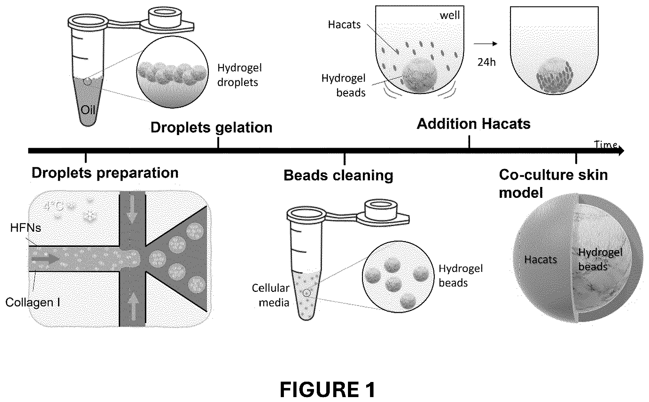

**Figure 1****: Sketch of the process to produce the spherical core-shell skin model.** The main steps of the process are the preparation of liquid collagen water-in-oil droplets in a microfluidic chip; droplet gelation into collagen beads via temperature increase; cleaning protocol to maintain cells viable while exchanging the surface affinity of the beads from oil to cellular media; and HACATs addition to the collagen beads.

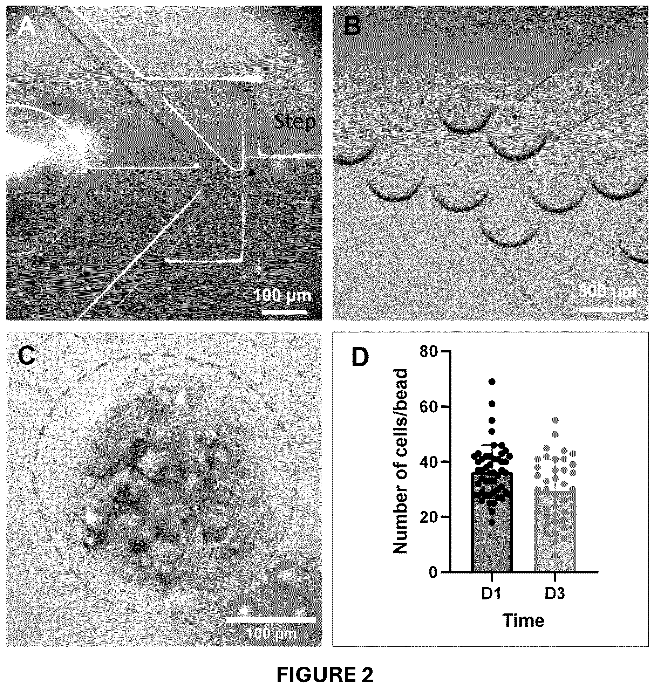

**Figure 2****: Collagen beads with embedded HFNs (CBs-HFNs).** A) Brightfield image of the flow focusing geometry with highlighted paths of oil and collagen + HFNs, and the step. **B)** Droplets of liquid collagen with HFNs in oil, in the collecting chamber of the microfluidic chip. C) Brightfield image of CBs-HFNs 11 hours after preparation. The edge of the CB is outlined with the dashed line. D) Histogram of the number of HFNs cells per collagen bead at D1 and D3.

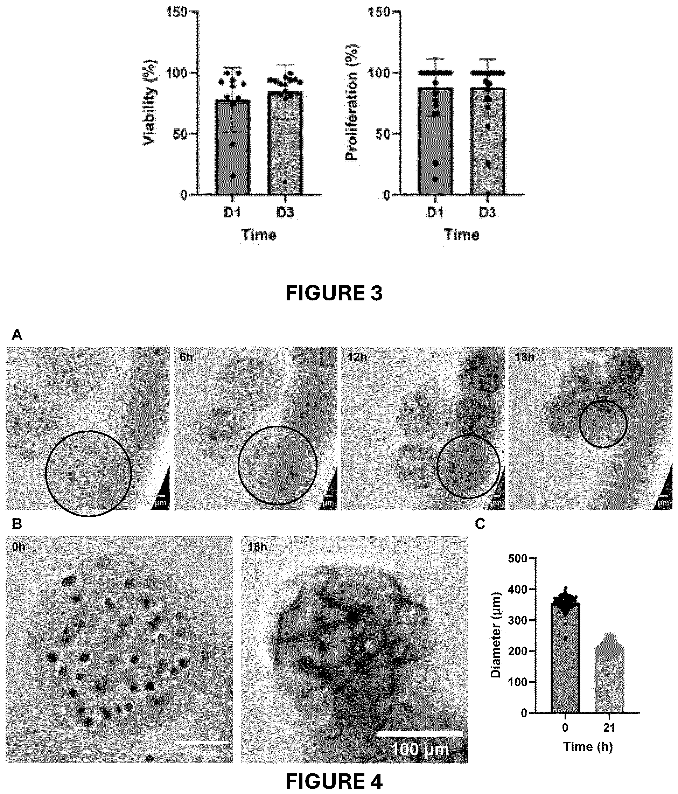

**Figure 3****: Evaluation of cellular viability and proliferation of CB-HFNs over 3 days *in vitro.*** Viability (DAPI/NucRed staining) and proliferation (DAPI/Ki67 staining) at D1 and D3 of CBs-HFNs.
**Figure 4****: Temporal evolution of CB-HFNs throughout the first 21 hours. A)** Bright field images of CBs-HFNs overtime during the first 18 hours after beads preparation. HFNs are free to move in the collagen matrix and they assemble the collagen fibers in a more compact manner. **B)** Bright field image of CB-HFNs, showing that the cells spread and adhere on the matrix. **C)** Final structure shrinks from an average diameter of 350 µm to 200 µm.

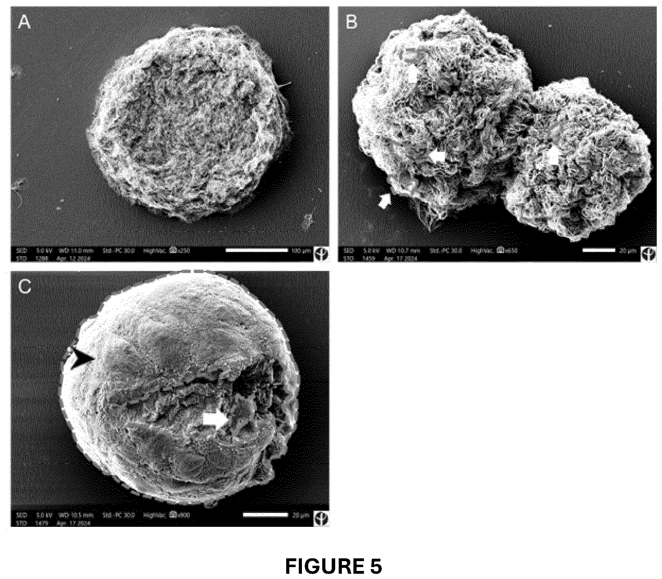

**Figure 5****: Electron microscopy characterization of the CB, CB-HFNs, and co-culture.** SEM images of respectively **A)** collagen beads, **B)** collagen beads with embedded HFNs (CB-HFNs), and C) collagen beads with HFNs inside and HACATs on the outside. In white and black arrows are respectively showed the areas where HFN and HACAT cells are located.

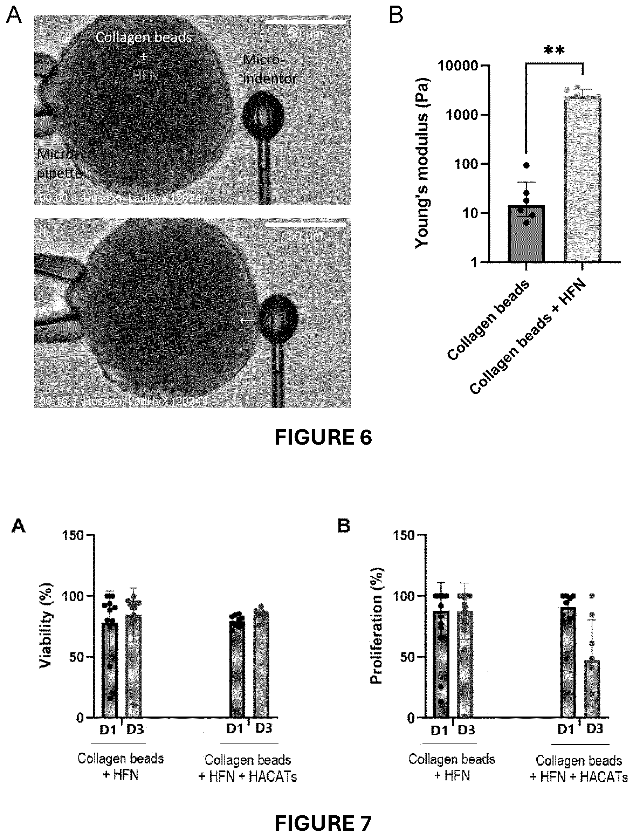

**Figure 6****: Stiffness measurements of CBs and CBs-HFNs by microindentation. A)** i. Brightfield microphotograph of the CB-HFNs held by the micropipette, next to the microindentor; ii. indentation of the CB-HFNs with the microindentor to measure its stiffness. **B)** Comparison of the Youngs modulus obtained for the CBs with and without HFNs.
**Figure 7****: Evaluation of cellular viability and proliferation of the co-culture over 3 days in vitro. A)** Viability and **B)** proliferation of the co-culture model compared to CB-HFNs.

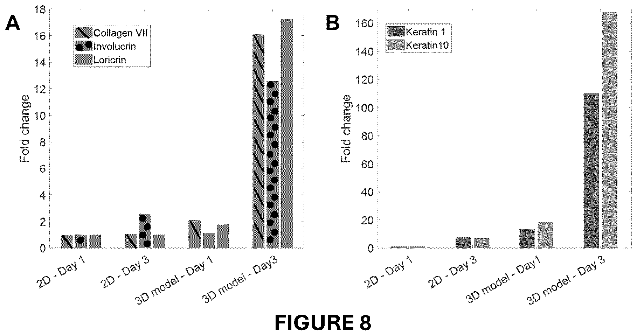

**Figure 8****: Time evolution of skin marker expression in 2D and 3D co-cultures. A)** Fold change expression of Collagen VII, Involucrin, and Loricrin compared to 2D, and B) Fold change expression of Keratin 1, and Keratin 10 compared to 2D.

### BRIEF DESCRIPTION OF THE SEQUENCES

**[Table 1]**

| **SEQ ID NO:** | **Type** | **Sequence** | **Description** |
|---|---|---|---|
| 1 | DNA | TGACAAGGTGAGGTTCCTGG | Forward primer for keratin 1 |
| 2 | DNA | GTTGGTCCACTCTCCTTCGG | Reverse primer for keratin 1 |
| 3 | DNA | TGATGTGAATGTGGAAATGAATGC | Forward primer for keratin 10 |
| 4 | DNA | GTAGTCAGTTCCTTGCTCTTTTCA | Reverse primer for keratin 10 |
| 5 | DNA | AAGGACCTCGGGGAGACAAT | Forward primer for collagen VII A1 |
| 6 | DNA | CGGATACCAGGCACTCCATC | Reverse primer for collagen VII A1 |
| 7 | DNA | CTGCCTCAGCCTTACTGTGA | Forward primer of involucrin |
| 8 | DNA | GGAGGAGGAACAGTCTTGAGG | Reverse primer for involucrin |
| 9 | DNA | GCAAACCTCGGGTAGCATCA | Forward primer for loricrin |
| 10 | DNA | GCCGTCCAAATAGATCCCCC | Reverse primer for loricrin |
| 11 | DNA | ATCAAGAAGGTGGTGAAGCAG | Forward primer for GAPDH |
| 12 | DNA | GTCGCTGTTGAAGTCAGAGG | Reverse primer for GAPDH |

### EXAMPLE

The present example describes the preparation of the spherical core-shell skin model of the invention.

### Materials and methods

### Cell culture preparation

Primary human fibroblasts (HFNs) harvested from surgical voluntary donations from young human foreskin (passage number between 4 and 8) and keratinocyte HACATs cell line (Cytion, #300493, passage number between 34 and 37) were cultured in T-75 cm² flasks (Corning, France), in a standard COzincubator (Binder, Tuttlingen, Germany) (5% (v/v) COz), using standard sterile cell culture procedures.

The culture medium was composed of Dulbecco's Modified Eagle's Medium (DMEM) (Gibco^{™}, Life Technologies, Saint Aubin, France) supplemented with 10% (v/v) fetal bovine serum (FBS) (Gibco^{™}) and 1% (v/v) penicillin-streptomycin (Gibco^{™}).

In total 5 × 10⁵ cells were seeded in a T75 flask and sub-cultivated every 3 days.

### Microfluidic protocol

A previously designed microfluidic device (disclosed in Sart et al. (2017) Nature Comm. 8:469 and international patent application WO2016/059302) was used to produce water-in-oil droplets (microdroplets) using a modified flow-focusing junction and step emulsification.

Molds with the design for the microfluidic device were created with dry resin Eternal Laminar^{®} E8013 (Eternal Materials, Taiwan) on glass slides. Poly(dimethylsiloxane) (PDMS, SYLGARD^{™} 184, Dow Corning) negative replicas of these molds were prepared with a liquid PDMS mix (10:1 w/w bulk to curing agent ratio) that was poured onto the mould and later baked at 65°C for at least 2 hours for solidification. Inlets and outlets were created with a 1 mm diameter hole-puncher and the PDMS slab was bonded to a glass slide using standard oxygen plasma bonding technique.

Before experiments, the microfluidic devices were surface-treated 3 times with Novec^{™}-1720 (3M, Paris, France), a fluoropolymer coating agent, for 30 min at 110° C on a hot plate.

To produce droplets, Novec^{™}-7500 Engineered Fluid (HFE) (3 M) with 3% (w/w) FluoroSurfactant (RAN Biotechnologies, Bervely, USA) was used as the continuous oil phase. The solutions were loaded in 1 mL glass syringes (SGE, Analytical Science, France) that were actuated with computer-controlled syringe pumps connected to the inlets of the PDMS device through polytetrafluoroethylene (PTFE) tubing.

Prior to every experiment, the microfluidic devices were first filled with the oil phase solution and kept at 4°C.

### Assembly of the stratified structure

HFNs were detached from the cell culture flasks using trypsin (Gibco^{™}). Cell concentration was determined using a hemocytometer. Cells were centrifuged at 1200 rpm for 5 min and resuspended at a concentration of 8 × 10⁶ cells/mL.

Collagen I high concentration from rat tail (354249, Corning) (concentration range 8-11 mg/ml) was prepared at 7 mg/mL on ice. Briefly, 60 µL of cold phosphate buffered saline (PSB) 10X and 9,38 µL of 1 M sodium hydroxide (NaOH) were added to 408 µL of collagen solution at 10.3 mg/mL and 123 µL of MiliQ water to obtain a collagen stock at 7 mg/mL. The pH of the solution was measured with a pHmeter to confirm a range between 6.3 and 7 before every batch preparation.

Once collagen was neutralized, 1 part of collagen and 2 parts of cells dispersed in culture medium were mixed to reach a final volume of 150 µL of solution and a final cell concentration of 2.3 × 10⁶ cells/mL.

One pre-chilled 1 ml glass syringe previously filled with HFE oil was loaded with the liquid collagen-HFNs solution. Aqueous droplets in oil were produced in the microfluidic device following the flow rates shown below.

**[Table 2]**

| **Solution** | **Liquid inlet** | **Flow rate (µL/min)** |
|---|---|---|
| Oil + surfactant | Chamber | 50 |
| Oil + surfactant | Junction | 50 |
| Hydrogel + cells | Aqueous phase inlet | 5 |

This operation was performed at 4°C, temperature at which all chips, syringes, connections and solutions were pre-chilled.

After 6 min of droplet production, about 2 × 10³ droplets (of 15 nL volume per droplet) were collected, and all flow rates were stopped. The droplets surrounded by oil were collected in a 1.5 mL Eppendorf tube.

Collagen droplets were gelled at 37°C for 10 min.

To clean the samples from oil, the fluorinated phase was replaced by a large volume of pure HFE. Then, 500 µL of an emulsion destabilization agent, 2% (v/v) 1H, 1H, 2H, 2H-perfluoro-1-octanol (PFO) solution dissolved in HFE and 500 µL of culture medium were added to the collagen beads-HFNs (CB-HFNs) pellet in this order.

The protocol yielded 1.2 × 10³ beads/ml, each bead containing about 40 HFNs.

CB-HFNs were then loaded in round bottom ultra-low adhesion 96-well plates at a concentration of 10 beads/mL *(i.e.* 1 bead per well).

Right after the CB-HFNs preparation, HACATs were detached from the culture flask using trypsin. After centrifugation, the pellet was resuspended at a final concentration of 4.5 × 10³ cells/mL in the culture medium. Then, the HACATs suspension was added to the previously loaded 96-well plates with the CB-HFNs (100 µL per well).

The plate was then kept for 1 h at 37°C in a CO₂ incubator on a rocker (PS-3D Sunflower mini-shaker, Grant-bio) at 8 rpm, to allow homogeneous distribution of HACATs on the beads.

After one day, HFNs reorganized the fibers in the collagen matrix, while HACATs formed an external tight layer on the outside of the structure.

### Staining of the co-culture

To assess cell viability, nucleic and dead cells were respectively stained with DAPI and NucRed Dead 647 ReadyProbes (Sigma-Aldrich and Thermo Fisher Scientific), following manufacturer's instructions.

Cell co-culture was imaged using a 10 mM solution of CellTracker Far Red and CFSE (Life Technologies) prepared in sterile Dimethyl sulfoxide (DMSO). HFNs and HACATs cells were respectively incubated for 30 min in culture medium with 10 µM CellTracker, followed by PBS washing.

Cell proliferation was assessed by immunostaining with rabbit polyclonal anti-Ki67 antibody (ab15580, Abcam) on fixed samples. Briefly, after an initial wash with PBS, samples were incubated for 40 min at room temperature in 4% (v/v) paraformaldehyde (PFA) (Alpha Aesar) in PBS. A second wash with PBS was then performed and the samples were incubated at room temperature for 30 min in 0.5% (v/v) Triton X-100 (Sigma-Aldrich) (PBS-t) to permeabilize the cell membrane. Samples were then incubated for 30 min at room temperature in 5% (v/v) FBS (PBS-f) for blocking. The primary antibody incubation was then performed at a ratio 1:100 in 1% PBS-f for 3 h at room temperature. Finally, after a PBS wash, the samples were incubated with anti-rabbit antibody from goat Alexa647 (1:100) in 1% PBS-f and DAPI, for 3 h at room temperature in the dark. The samples were washed with PBS twice before imaging.

### Image acquisition

Fluorescence images were acquired using an inverted microscope (Eclipse Ti, Nikon), equipped with a motorized stage, an illumination system (Spectra-X, Lumencor), a temperature-controlled incubator (Okolab, Pozzuoli, Italy), and a CMOS camera (ORCA Flash 4.0, Hamamatsu).

3D skin models were imaged through a z-stack in confocal mode. Z-steps of 5-10 µm were chosen to scan about 120 µm of the sample.

### Image analysis

Stratified structures were detected using bright field microscopy. Cells were detected suing bright field and fluorescence confocal imaging.

Cell viability was determined as follows: after DAPI and NucRed live staining, a max intensity projection of all the planes imaged was performed on the FIJI image processing package. Then, local background was used for each image to determine a specific threshold for the fluorescent dead cells and nuclear staining with DAPI. Viability was then defined as viability = 1 - (N/A), where N and A are respectively the number of dead pixels and the area of the DAPI staining.

Cell proliferation was estimated as the ratio between Ki67-positive and DAPI-positive detected areas per bead on the FIJI image processing package. The number of cells per bead was obtained after intensity projection on the DAPI fluorescent signal, using the TrackMate plugin with the following parameters: estimated object diameter: 8 µm, quality threshold: 0.2.

### RT-qPCR analysis

Total RNAs were extracted and purified using the RNeasy micro kit (Qiagen). 100 ng of RNA were reverse transcribed using z Quantitect Reverse Transcription kit (Qiagen). cDNAs were amplified using a FastStart Universal SYBR Green Master Mix (containing Rox) (Roche) and primers (Eurofins Scientific, France) summarized in the table below, at the specified melting temperature (Tm) using a QuantStudio 3 (Thermo Fisher Scientific) thermocycler.

**[Table 3]**

| **Name** | **Orientation** | **Sequence** | **Size (bp)** | **Tm (°C)** |
|---|---|---|---|---|
| Keratin 1 | Forward | TGACAAGGTGAGGTTCCTGG (SEQ ID NO: 1) | 149 | 60 |
| | Reverse | GTTGGTCCACTCTCCTTCGG (SEQ ID NO: 2) | | |
| Keratin 10 | Forward | TGATGTGAATGTGGAAATGAATGC (SEQ ID NO: 3) | 147 | 58 |
| | Reverse | GTAGTCAGTTCCTTGCTCTTTTCA (SEQ ID NO: 4) | | |
| Collagen VII A1 | Forward | AAGGACCTCGGGGAGACAAT (SEQ ID NO: 5) | 181 | 60 |
| | Reverse | CGGATACCAGGCACTCCATC (SEQ ID NO: 6) | | |
| Involucrin | Forward | CTGCCTCAGCCTTACTGTGA (SEQ ID NO: 7) | 121 | 60 |
| | Reverse | GGAGGAGGAACAGTCTTGAGG (SEQ ID NO: 8) | | |
| Loricrin | Forward | GCAAACCTCGGGTAGCATCA (SEQ ID NO: 9) | 106 | 60 |
| | Reverse | GCCGTCCAAATAGATCCCCC (SEQ ID NO: 10) | | |
| GAPDH | Forward | ATCAAGAAGGTGGTGAAGCAG (SEQ ID NO: 11) | 96 | 58 |
| | Reverse | GTCGCTGTTGAAGTCAGAGG (SEQ ID NO: 12) | | |

As a negative control, water and total RNA served as template for PCR.

To validate specificity of the PCR, amplicons were analysed by dissociation curve. Change in gene expression was calculated using the 2^{-ΔΔCt} method, using GAPDH as housekeeping gene.

### Results

To produce the spherical core-shell skin models, a previously designed droplet microfluidic platform was used to build the inner core of the 3D structure. This core was composed of a collagen bead with embedded HFNs. In the second stage of the method, carried out off-chip, HACATs were added on top of the core structure to form an outer shell.

This stratified structure partially mimics the natural organization of human skin. HFNs within the collagen matrix mimic the dermis-equivalent part of the skin, while the external tight layer of HACATs model epidermis. Thereby, the epidermis-like layer, on top of the dermis-like compartment, can directly be exposed to external agents added to the culture medium (*e.g.* cosmetic ingredients, etc...) or particular conditions (*e.g* environmental stress, etc...).

The main steps of the preparation method are depicted in Figure 1.

Using a microfluidic chip equipped with a flow-focuser allowed the formation of monodispersed micro-CBs-HFNs of a diameter of about 300 µm, which contained an average of 40 HFNs per bead **(****Figure 2****).** Cell concentration in the collagen solution, thus the final number of cells per bead, was selected depending on the final application in the chip. In the present case, the final HFN concentration in the collagen beads (2.3 × 10⁶ cells/mL) is within the same range as the cell concentration generally used in bioprinting (generally around 1.6 × 10⁶ cells/mL) for dermis reconstruction.

Noteworthy, it was advantageous to reach an equilibrium value between providing enough cells to build a complete model after 1 day *in vitro* and obtaining a final number of cells which do not deplete the nutrients present in the droplet in a shorter time frame than the duration of the experiment.

Viability and proliferation of HFNs in the CBs was assessed. The resulted are reported in Figure 3. HFNs viability was over 75% both 1 day (D1) and 3 days (D3) *in vitro* after preparation. Additionally, cells exhibited a proliferation of over 80% during the investigated period.

HFNs remodelled collagen organization overtime. **Figure 4** shows the contraction of a collagen bead in the first 18 hours after CB-HFNs preparation. The structures decreased in size (from an average of 350 µm to 200 µm) because of the pulling of HFNs on collagen fibers mesh.

This remodelling was investigated through Scanning Electron Microscopy (SEM) measurements of CB-HFNs. A collagen bead prepared without HFNs is shown in **Figure 5A****.** Randon arrangement of collagen fibers in this structure was substituted by a more organized pattern in the presence of HFNs as shown in **Figure 5B****.**

This behaviour also influenced stiffness of CB-HFNs. To explore the role of HFNs in regulating collagen matrix rigidity, Young's modulus measurements were performed on CBs with and without HFNs, using micro indentation. The results, showed in **Figure 6****,** indicate that there is a major change in stiffness of about 100 orders of magnitude between CBs without HFNs (14 Pa) and CB-HFNs (2.4 kPa). Advantageously, the estimated stiffness of CB-HFNs is close to the stiffness range generally reported for skin (5 kPa - 140 MPa). These results suggest that remodelling of collagen fibers induced by HFNs increases rigidity of collagen beads up to a value that is close to dermal equivalents.

In the last step of the co-culture assembly, HACATs were added to CB-HFNs to maintain a final number of HACAT cells lower than 0.5 × 10⁶ cells/mL. The ratio between the number of HFNs and HACATs (namely 1:4) in the 3D structures was adapted from the literature using 2D skin models.

Confocal images of CB-HFNs showed that HACATs colonized CB-HFNs and formed a uniform layer on the outside of the beads. A thickness of about 20 µm of HACATs signal surrounded the CB-HFNs core in all directions investigated. In other words, at least a double layer of HACATs cells was obtained on the outside of CB-HFNs. This was also visible in SEM images. Rigidity of the dermal model allowed HACATs to adhere and build a tight and compact layer on the outside of the collagen beads, that resembles the epidermis layer, as visible in **Figure 5C****.**

A core-shell structure that mimicked structural organization of skin was thus obtained. This spatial organization was maintained overtime (up to 7 days) and cells were viable and proliferative over 3 days, as shown in **Figure 7****.**

Surprisingly, this core-shell structure could not be obtained using any type of gelling agent to form the beads. Indeed, the inventors tested several types of gelling agents. They showed that, for example, using Matrigel^{®} instead of collagen, HFNs were able to spread on the matrix but HACATs formed independent clusters and could not form a layer around the core. On the contrary, using collagen allowed both the survival of encapsulated HFNs and the adhesion of HACATs layer around it.

Keratinocytes phenotype was evaluated by RT-qPCR analysis. As shown in **Figure 8****,** cells re-expressed some key skin phenotypic markers in the 3D model, in comparison with 2D cultures. For instance, increased expression of collagen VII showed that interaction between HFN and HACATs increased after 3 days in the 3D model. Similarly, increased expression of involucrin and loricrin after 3 days *in vitro* suggested the formation of an epidermal barrier in the 3D model. Moreover, increased expression of keratin 1 and keratin 10 after 3 days *in vitro* indicated that HACATs phenotype in the 3D model resembled the spinous and granular layers of epidermis.

Together, these results show that droplet microfluidics enabled the production of a functional 3D skin *in vitro* model.

### REFERENCES

- Anna et al. (2003) Formation of dispersions using « flow focusing » in microchannels. Appl. Phys. Lett. 82:364.
- Sart et al. (2017) Multiscale cytometry and regulation of 3D cell cultures on a chip. Nature Communications 8:469
- Thorsen et al. (2001) Dynamic pattern formation in a vesicle-generating microfluidic device. Phys. Rev. Lett. 86:4163-4166.
- FR2958186 (Ecole Polytechnique) - Dispositif de formation de gouttes dans un circuit microfluide.
- WO2016/059302 (Ecole Polytechnique; Centre National de la Recherche Scientifique) - Method for handling microdrops which include samples.
- US2011/0306110 (The University of Tokyo) - Method for three-dimensional hierarchical cell co-culture.

## Claims

1. A method for preparing a spherical core-shell skin model, said method comprising the steps of:
a) forming microdroplets of an aqueous solution comprising fibroblasts and collagen, in an oil, preferably at a temperature comprised between 2°C and 10°C, more preferably at a temperature of 4°C, wherein the formed collagen microdroplets comprise at least one fibroblast per microdroplet, preferably between 2 × 10⁶ and 3 × 10⁶ fibroblasts/mL of collagen microdroplet;
b) gelling at least a portion of the formed collagen microdroplets comprising said at least one fibroblast, thereby obtaining collagen beads comprising at least one fibroblast per bead, preferably between 2 × 10⁶ and 3 × 10⁶ fibroblasts/mL of collagen bead;
c) replacing the oil surrounding the collagen beads comprising said at least one fibroblast with a culture medium,
d) contacting (i) the collagen beads comprising said at least one fibroblast with (ii) keratinocytes, preferably at a ratio number of fibroblasts : number of keratinocytes comprised between 1:2 and 1:8, more preferably at a ratio number of fibroblasts : number of keratinocytes of 1:4, and
e) incubating (i) said collagen beads with (ii) said keratinocytes for a period of time enabling the formation of an external layer by said keratinocytes around said collagen beads, thereby obtaining a spherical core-shell skin model, the core comprising at least one fibroblast and the shell comprising at least one keratinocyte.

2. The method according to claim 1, wherein step a) is implemented using a microfluidic device and comprises:
a1) injecting, into a zone of said microfluidic device, said aqueous solution comprising fibroblasts and collagen,
a2) injecting said oil into said zone of said microfluidic device, in order to drive the aqueous solution comprising fibroblasts and collagen towards an outlet of said microfluidic device, the oil being injected so as to form collagen microdroplets comprising at least one fibroblast per microdroplet, preferably between 2 × 10⁶ and 3 × 10⁶ fibroblasts/mL of collagen microdroplet, and
a3) collecting, from said microfluidic device, said formed collagen microdroplets comprising at least one fibroblast per microdroplet.

3. The method according to claim 1 or 2, wherein said collagen is present in said aqueous solution at a concentration of 4 mg/mL to 10 mg/mL, preferably at a concentration of 5 mg/mL.

4. The method according to any one of claims 1 to 3, wherein said fibroblasts are present in said aqueous solution at a concentration of 2 × 10⁶ cells/mL to 3 × 10⁶ cells/mL.

5. The method according to any one of claims 1 to 4, wherein said oil comprises a surfactant, preferably a fluorinated surfactant.

6. The method according to any one of claims 1 to 5, wherein the gelling step b) comprises heating said formed collagen microdroplets, preferably at a temperature of 37° C.

7. The method according to any one of claims 1 to 6, comprising, before the replacing step c), a step of cleaning the gelled collagen beads from oil, preferably by contacting the collagen beads with an emulsion destabilization agent.

8. The method according to any one of claims 1 to 7, wherein, in step d), the collagen beads (i) are contacted with a keratinocytes suspension (ii) at a concentration of 4 × 10¹ keratinocytes/mL to 5 × 10¹ keratinocytes/mL.

9. Spherical core-shell skin model obtainable by the method according to any one of claims 1 to 8.

10. Spherical core-shell skin model comprising:
- a core comprising fibroblasts in a collagen matrix, and
- a shell, totally or partially surrounding said core, the shell comprising keratinocytes, preferably a double layer, or more, of keratinocytes, forming an external layer on the outside of said core,
wherein the spherical core-shell skin model preferably has a diameter of from 100 µm to 350 µm.

11. The spherical core-shell skin model according to claim 10, having a stiffness of more than 2 kPa.

12. The spherical core-shell skin model according to claim 10 or 11, wherein said keratinocytes display a higher expression level of collagen VII, involucrin, loricrin, keratin 1 and/or keratin 10 compared to the same cells cultured, alone or in co-culture with fibroblasts, in two-dimensions, typically on flasks.

13. Use of the spherical core-shell skin model according to any one of claims 9 to 12 for screening cosmetically, dermatologically or pharmaceutically active candidate compounds.

14. A method for screening cosmetically, dermatologically or pharmaceutically active candidate compounds, said method comprising the steps of:
- providing at least one spherical core-shell skin model according to any one of claims 9 to 12, and
- contacting said at least one spherical core-shell skin model with at least one cosmetically, dermatologically or pharmaceutically active candidate compound.

15. Use of the spherical core-shell skin model according to any one of claims 9 to 12 as a research tool for evaluating and/or monitoring the effect of external conditions on the expression level of skin cells proteins and/or on the mechanical properties of skin.
